# EUROPEAN PATENT APPLICATION

(11) **EP 1 160 572 A1**
(43) Date of publication of application: **05.12.2001**
(21) Application number: 00906685.3
(22) Date of filing: 03.03.2000
(51) Int. Cl.: G01N 33/53, A61K 39/395, A61K 51/10

(54) **DIAGNOSTICS AND REMEDIES FOR LEUKEMIA**

(30) Priority: 04.03.1999 JP 5637999
(71) Applicant: KYOWA HAKKO KOGYO CO., LTD., Chiyoda-ku, Tokyo 100-8185 (JP)
(72) Inventor: SHITARA, Kenya Kyowa Hakko Kogyo Co., Ltd., Machida-shi Tokyo 194-8533 (JP); SHIBUYA, Masabumi, Kawaguchi-shi Saitama 333-0866 (JP)
(74) Representative: Kinzebach, Werner, Dr.
(86) International application number: JP0001294
(87) International publication number: WO0052470

(57) **Abstract**

The present invention relates to treating and diagnosing leukemia using an anti-human VEGF receptor Flt-1 antibody, and a diagnostic agent and a therapeutic agent comprising the anti-human VEGF receptor Flt-1 antibody as an active ingredient.

## Description

### TECHNICAL FIELD

The present invention relates to a method for diagnosing leukemia using an anti-human VEGF receptor Flt-1 antibody. Also, it relates to a diagnostic agent and a therapeutic agent comprising the anti-human VEGF receptor Flt-1 antibody as an active ingredient.

### BACKGROUND ART

Angiogenesis plays an important role in the formation of the circulatory organ system and construction of many tissues in the embryonal period of vertebrates and also closely relates to the formation of corpus luteum during sexual cycle, transient proliferation of uterine endometrium, formation of placenta and the like in mature individuals (females). With regard to pathological states, angiogenesis closely relates to the proliferation or metastasis of solid tumors and formation or acceleration of morbid states of diabetic retinopathy and rheumatoid arthritis (*J. Biol*. *Chem.*, 267, 10931 (1992)). Vascular permeability factor (VPF)/vascular endothelial growth factor (VEGF) are known as the most important factors involved in angiogenesis induction, such as during the developmental and pathological conditions described above (*Advances in Cancer Research,* 67, 281 (1995)).

Among diseases accompanied by angiogenesis, it has been reported that VEGF closely relates to the proliferation or metastasis of solid tumors and formation of morbid states of diabetic retinopathy and rheumatoid arthritis. With regard to solid tumors, it has been reported that VEGF is produced in a number of human tumor tissues, such as renal carcinoma (*Cancer Research*, 54, 4233 (1994)), breast cancer (*Human Pathology*, 26, 86 (1995)), brain tumor (*J. Clinical Investigation*, 91, 153 (1993)), digestive organ cancer (*Cancer Research*, 53, 4727 (1993)), ovarian cancer (*Cancer Research*, 54, 276 (1994)) and the like.

Two kinds of human VEGF receptor have been reported, namely a first receptor Flt-1 (fms-like tyrosine kinase) (*Oncogene*, 5, 519 (1990); *Science*, 255, 989 (1992)) and a second receptor KDR (kinase insert domain-containing receptor) (WO 92/14748, Priority Feb. 22, 1991; *Biochem*. *Biophys. Res. Comm.*, 187, 1579 (1992)), which belong to the receptor-type tyrosine kinase family. A mouse-type homologue of the human-type VEGF receptor KDR is named Flk-1 (*Proc. Natl. Acad. Science, USA*, 88, 9026 (1991); WO 94/11499, Priority Nov. 13, 1992; *Cell*, 72, 835 (1993)). In the Flt-1 and KDR/Flk-1, the extracellular domain is composed of seven immunoglobulin-like domains, and the intracellular domain is composed of a membrane protein of 180 to 200 kilodalton in molecular weight which has a tyrosine kinase region. VEGF specifically binds to Flt-1 and KDR/Flk-1 at KD values of 20 pM and 75 pM, respectively. It has been reported that Flt-1 and KDR/Flk-1 are expressed specifically in vascular endothelial cells (*Proc. Natl. Acad. Science, USA*, 90, 7533 (1993); *Proc. Natl. Acad*. *Science, USA*, 90, 8915 (1993)). It has been reported that Flt-1 is expressed in human monocyte and relates to the migration of cells (*Blood*, 87, 3336 (1996)).

With regard to the expression of Flt-1 in various diseases, it has been reported that the expression of flt-1 mRNA is more highly increased in tumor vascular endothelial cells of human glioblastoma tissues (*Nature*, 359, 845 (1992)) and tumor vascular endothelial cells of human digestive organ cancer tissues (*Cancer Research*, 53, 4727 (1993)) than vascular endothelial cells in normal tissues. In addition, it has been reported that the expression of flt-1 mRNA is also observed in vascular endothelial cells of joints of patients with rheumatoid arthritis by *in situ* hybridization (*Journal Experimental Medicine*, 180, 341 (1994)). These results strongly suggest that a VEGF-VEGF receptor-Flt-1 system plays an important role in tumor angiogenesis. Although it has been reported that VEGF binds to Flt-1 and the intracellular domain is autophosphorylated (*Science*, 255, 989 (1992)), detailed functions are unknown. However, flt-1 knock out mouse in which the flt-1 gene had been destroyed, the animal died after a fetal age of 8.5 to 9.5 days due to abnormal blood vessel construction caused by abnormal morphology of .vascular endothelial cells during the blood island formation in the early stage of development and subsequent angiogenesis, it has been assumed that Flt-1 functions in a manner essential for the hollow tube formation of vascular endothelial cells in angiogenesis (*Nature*, 376, 66 (1995)).

Thus, although it has been reported that VEGF and VEGF receptor Flt-1 relate to the proliferation or metastasis of solid tumors, nothing has so far been reported on their relation to leukemia.

### DISCLOSURE OF THE INVENTION

A useful method for diagnosing and treating leukemia which is a disease caused by the tumorigenic change of a hematopoietic cell is desired.

The present inventors have found for the first time that an antibody against the VEGF receptor Flt-1 reacts with a leukemia cell line at a high ratio and thereby accomplished the present invention. That is, the present invention relates to the following (1) to (18).
(1) A diagnostic agent for leukemia, comprising an anti-human VEGF receptor Flt-1 antibody as an active ingredient.
(2) The diagnostic agent according to (1), wherein the anti-human VEGF receptor Flt-1 antibody is a monoclonal antibody.
(3) The diagnostic agent according to (1), wherein the anti-human VEGF receptor Flt-1 antibody is an antibody selected from the group consisting of KM1730, KM1731, KM1732, KM1748 and KM1750.
(4) The diagnostic agent according to (1), wherein the anti-human VEGF receptor Flt-1 antibody is a humanized antibody.
(5) The diagnostic agent according to (1), wherein the anti-human VEGF receptor Flt-1 antibody is an antibody selected from the group consisting of Fab, Fab', F(ab')₂, a single chain antibody and a disulfide stabilized antibody.
(6) The diagnostic agent according to (1), wherein the anti-human VEGF receptor Flt-1 antibody is an antibody fused with a radioisotope, a protein or a low molecular weight agent by a chemical or genetic engineering means.
(7) A therapeutic agent for leukemia, comprising an anti-human VEGF receptor Flt-1 antibody as an active ingredient.
(8) The therapeutic agent according to (7), wherein the anti-human VEGF receptor Flt-1 antibody is a monoclonal antibody.
(9) The therapeutic agent according to (7), wherein the anti-human VEGF receptor Flt-1 antibody is an antibody selected from the group consisting of KM1730, KM1731, KM1732, KM1748 and KM1750.
(10) The therapeutic agent according to (7), wherein the anti-human VEGF receptor Flt-1 antibody is a humanized antibody.
(11) The therapeutic agent according to (7), wherein the anti-human VEGF receptor Flt-1 antibody is an antibody selected from the group consisting of Fab, Fab', F(ab')₂, a single chain antibody and a disulfide stabilized antibody.
(12) The therapeutic agent according to (7), wherein the anti-human VEGF receptor Flt-1 antibody is an antibody fused with a radioisotope, a protein or a low molecular weight agent by a chemical or genetic engineering means.
(13) A method for diagnosing leukemia, comprising using an anti-human VEGF receptor Flt-1 antibody.
(14) The method for diagnosing leukemia according to (13), wherein the anti-human VEGF receptor Flt-1 antibody is a monoclonal antibody.
(15) The method for diagnosing leukemia according to (13), wherein the anti-human VEGF receptor Flt-1 antibody is an antibody selected from the group consisting of KM1730, KM1731, KM1732, KM1748 and KM1750.
(16) The method for diagnosing leukemia according to (13), wherein the anti-human VEGF receptor Flt-1 antibody is a humanized antibody.
(17) The method for diagnosing leukemia according to (13), wherein the anti-human VEGF receptor Flt-1 antibody is an antibody selected from the group consisting of Fab, Fab', F(ab')₂, a single chain antibody and a disulfide stabilized antibody.
(18) The method for diagnosing leukemia according to (13), wherein the anti-human VEGF receptor Flt-1 antibody is an antibody fused with a radioisotope, a protein or a low molecular weight agent by a chemical or genetic engineering means.

The antibody used in the present invention may be any antibody, so long as it can bind to the VEGF receptor Flt-1 (hereinafter simply referred to as "Flt-1") and has a diagnostic or therapeutic effect on leukemia. The anti-Flt-1 antibody used in the present invention can be obtained as a polyclonal or monoclonal antibody using conventionally known means (*Antibodies-A Laboratory Manual*, Cold Spring Harbor Laboratory, 1988 (hereinafter referred to as "*Antibodies-A Laboratory Manual*")).

As the anti-Flt-1 antibody used in the present invention, any of polyclonal and monoclonal antibodies can be used, but a monoclonal antibody derived from a mammal is preferred.

The monoclonal antibody derived from a mammal includes an antibody produced by a hybridoma, a gene recombinant antibody produced by a transformant transformed with an expression vector containing an antibody gene, and a human antibody.

Preferred examples of the monoclonal antibody used in the present invention include those which are established by the following production method.

Examples include an anti-human VEGF receptor Flt-1 monoclonal antibody obtained by preparing a human VEGF receptor Flt-1 protein as an antigen, inducing a plasma cell having the antigen specificity from an animal immunized with the antigen, fusing the cell with a myeloma cell to thereby prepare a hybridoma, culturing the hybridoma or administering the hybridoma to a mammal to cause ascites tumor, and then separating and purifying the culture or ascitic fluid.

The gene recombinant antibody used in the present invention includes those in which the above monoclonal antibody of the present invention is modified using gene recombination techniques. Examples include a humanized antibody, an antibody fragment and the like. Among these antibodies, an antibody having low antigenicity and prolonged blood half-life is preferred as a therapeutic agent.

The humanized antibody used in the present invention includes a human chimeric antibody and a human CDR (complementary determining region; hereinafter referred to as "CDR")-grafted antibody.

The human chimeric antibody means an antibody containing an antibody variable region heavy chain (hereinafter referred to as "VH"), a variable region light chain (hereinafter referred to as "VL") of an animal other than human, a constant region heavy chain (hereinafter referred to as "CH") of a human antibody, and a constant region light chain (hereinafter referred to as "CL") of a human antibody.

The human chimeric antibody used in the present invention can be produced by preparing cDNAs encoding VH and VL from a hybridoma capable of producing a monoclonal antibody which binds to the human VEGF receptor Flt-1, inserting each of them into an expression vector for animal cell containing genes encoding human antibody CH and human antibody CL to thereby construct a human chimeric antibody expression vector, and then introducing the vector into an animal cell to thereby express the antibody.

A human CDR-grafted antibody is an antibody in which CDR sequences of VH and VL of a human antibody are replaced by respective CDR sequences of an antibody of an animal other than human.

A human CDR-grafted antibody used in the present invention can be produced by constructing cDNAs encoding V regions in which the VH and VL CDR sequences of an optional human antibody are replaced by the VH and VL CDR sequences of an antibody of an animal other than human, respectively, which binds to the human VEGF receptor Flt-1, inserting each of them into an expression vector for animal cell containing a genes encoding human antibody CH and human antibody CL to thereby construct a human CDR-grafted antibody expression vector, and then introducing the vector into an animal cell to express the antibody.

The human antibody moiety of the human chimeric antibody and human CDR-grafted antibody used in the present invention may belong to any immunoglobulin (Ig) class, but an IgG-type is preferred, and any C region of immunoglobulin belonging to the IgG-type (e.g., IgG1, IgG2, IgG3, IgG4 or the like) can be used.

A human antibody is an antibody naturally existing in the human body, as well as an antibody obtained from a human antibody phage library and a human antibody-producing transgenic animal, which are prepared based on the recent advance in genetic engineering, cell engineering and developmental engineering.

Regarding the antibody existing in the human body, a lymphocyte capable of producing the antibody can be cultured by isolating a human peripheral blood lymphocyte, immortalizing it by infection with EB virus or the like and then cloning it, and the antibody can be purified from the culture.

A human antibody phage library is a library in which antibody fragments such as Fab, a single chain antibody and the like are expressed on the phage surface by inserting an antibody gene prepared from human B cell into a phage gene. A phage expressing an antibody fragment having the desired antigen binding activity can be recovered from the library, using its activity to bind to an antigen-immobilized substrate as the marker. The antibody fragment can be further converted into a human antibody molecule containing two complete H chains and two complete L chains by genetic engineering techniques.

The human antibody-producing transgenic animal is an animal in which a gene encoding a human antibody has been introduced into a cell. A human antibody-producing transgenic animal can be produced by introducing a gene encoding a human antibody into a mouse ES cell, transplanting the ES cell into an early stage embryo of other mouse and then developing the animal. Regarding the production method of a human antibody from the human antibody-producing transgenic animal, a human antibody-producing hybridoma is obtained by a hybridoma production method usually carried out in mammals other than human, and then culturing it to thereby produce and accumulate the human antibody in the culture.

An antibody fragment used in the present invention includes Fab (abbreviation of "fragment of antigen binding"), Fab', F(ab')₂, a single chain antibody (single chain Fv; hereinafter referred to as "scFv") and a disulfide stabilized antibody (disulfide stabilized Fv; hereinafter referred to as "dsFv"), which are antibody fragments having the affinity for the human VEGF receptor Flt-1.

A Fab is a fragment of about 50,000 molecular weight having an antigen binding activity, which is constituted by about half of the N-terminal side of H chain obtained by digesting the upper side of two disulfide bonds cross-linking two H chains in the hinge regions of IgG with papain, and the L chain.

The Fab used in the present invention can be obtained by treating the anti-human VEGF receptor Flt-1 antibody with papain. Alternatively, the Fab can be produced by inserting DNA encoding a Fab fragment of the anti-human VEGF receptor Fit-1 antibody into an expression vector for animal cells, and then expressing the antibody by introducing the vector into an animal cell.

The Fab' is a fragment of about 50,000 in molecular weight having an antigen binding activity, which is obtained by cutting the disulfide bonds between the hinges of the above F(ab')₂.

The Fab' used in the present invention can be obtained by treating the anti-human VEGF receptor Flt-1 antibody with a reducing agent, dithiothreitol. Alternatively, the Fab' can be produced by inserting DNA encoding a Fab' fragment of the anti-human VEGF receptor Flt-1 antibody into an expression vector for animal cells, and then expressing the fragment by introducing the vector into an animal cell.

The F(ab')₂ is a fragment of about 100,000 molecular weight having an antigen binding activity, which is constituted by two bound Fab regions at the hinge part which are obtained by digesting the lower side of two disulfide bonds in the hinge regions of IgG with trypsin.

The F(ab')₂ used in the present invention can be obtained by treating the anti-human VEGF receptor Flt-1 antibody with trypsin. Alternatively, the F(ab')₂ can be produced by inserting DNA encoding a F(ab')₂ fragment of the anti-human VEGF receptor Flt-1 antibody into an expression vector for animal cells, and then expressing the fragment by introducing the vector into an animal cell.

The single chain antibody (scFv) is a VH-P-VL or VL-P-VH polypeptide obtained by linking a VH chain and a VL chain using an appropriate peptide linker (hereinafter referred to as "P"). As the VH and VL contained in the scFv used in the present invention, any of the anti-human VEGF receptor Flt-1 antibody and human CDR-grafted antibody can be used.

The single chain antibody used in the present invention can be produced by obtaining cDNA encoding VH and VL from a hybridoma capable of producing an antibody which binds to the human VEGF receptor Flt-1, constructing a single chain antibody expression vector therefrom and then expressing it by introducing the vector into *Escherichia coli*, yeast, an animal cell or an insect cell.

The disulfide stabilized antibody (dsFv) is an antibody in which polypeptides prepared by replacing an amino acid residue in each of VH and VL with a cysteine residue are linked via a disulfide bond. The amino acid residue to be replaced by a cysteine residue can be selected based on the three-dimensional structure estimation of the antibody in accordance with the method shown by Reiter *et al.* (*Protein Engineering, 1*, 697 (1994)). As the VH or VL contained in the disulfide stabilized antibody used in the present invention, any of the mouse anti-human VEGF receptor Flt-1 monoclonal antibody and human CDR-grafted antibody can be used.

The disulfide stabilized antibody used in the present invention can be produced by obtaining cDNA encoding VH and VL from a hybridoma capable of producing an antibody which reacts with the human VEGF receptor Flt-1, inserting the cDNA into an appropriate expression vector and then expressing the antibody by introducing the expression vector into *Escherichia coli*, yeast, an animal cell or an insect cell.

A fusion antibody is an antibody described herein fused with a radioisotope, a protein, a low molecular weight agent or the like by a chemical or genetic engineering means.

The fusion antibody used in the present invention can be produced by chemically fusing an antibody which binds to the human VEGF receptor Flt-1 with a radioisotope, a protein, a low molecular weight agent or the like. Also, a fusion antibody with a protein can be produced by linking cDNA encoding the antibody to cDNA encoding the protein, inserting it into an appropriate expression vector and then expressing it by introducing the expression vector into *Escherichia coli*, yeast, an animal cell or an insect cell.

### 1. Production method of anti-human VEGF receptor Flt-1 monoclonal antibody

### (1) Preparation of antigen

Examples of the antigen necessary for preparing the anti-human VEGF receptor Flt-1 monoclonal antibody include cells in which the human VEGF receptor Flt-1 has been expressed on the cell surface or a cell membrane fraction thereof, a soluble human VEGF receptor Flt-1 protein having an extracellular region of different amino acid length, a fusion protein of the protein with the Fc region of the antibody, and the like.

The cells capable of expressing the human VEGF receptor Flt-1 on the cell surface include NIH3T3-Flt-1 cell (*Cell Growth & Differentiation,* 7, 213 (1996)). The full length or a partial fragment of the human VEGF receptor Flt-1 can be produced inside the cells or in a culture supernatant as such or as a fusion protein, by constructing a recombinant vector in which cDNA encoding the full length or a partial fragment of the human VEGF receptor Flt-1 (*Cell Growth & Differentiation, 1*, 213 (1996)) is inserted into the downstream site of the promoter of an appropriate vector, introducing the vector into a host cell, and then culturing the thus obtained human VEGF receptor Flt-1-expressing cells in an appropriate medium. Alternatively, it can also be prepared by synthesizing a polypeptide having a partial sequence of the above protein using an amino acid synthesizer.

Any host such as bacteria, yeast, animal cells, insect cells and the like can be used, so long as they can express the gene of interest. Examples of the bacteria include bacteria belonging to the genera *Escherichia, Bacillus* and the like such as *Escherichia coli, Bacillus subtilis* and the like. Examples of the yeast include *Saccharomyces cerevisiae, Schizosaccharomyces pombe* and the like. Examples of the animal cells include namalwa cell as a human cell, COS cell as a monkey cell, CHO cell as a Chinese hamster cell and the like. Examples of the insect cells include Sf9 and Sf21 (manufactured by Pharmingen), High Five (manufactured by Invitrogen) and the like.

As the vector for introducing DNA encoding Flt-1, any vector can be used, so long as the DNA can be introduced therein and expressed in a host cell.

When a bacterium such as *Escherichia coli* is used as the host, the expression vector may be preferably constructed with a promoter, a ribosome binding sequence, the DNA of the present invention, a transcription termination sequence and optionally a promoter controlling sequence. Examples include commercially available pGEX (manufactured by Pharmacia), pET System (manufactured by Novagen) and the like.

As the method for introducing the recombinant vector into a bacterium, any one of the known methods for introducing DNA into bacteria, such as a method in which a calcium ion is used (*Proc. Natl. Acad. Sci., USA*, 69, 2110 (1972)), a protoplast method (Japanese Published Unexamined Patent Application No. 2483942/88) and the like, can be used.

When yeast is used as the host, YEp13 (ATCC 37115), YEp24 (ATCC 37051), YCp50 (ATCC 37419) or the like may be used as the expression vector.

As the method for introducing the recombinant vector into yeast, any one of the known methods for introducing DNA into yeast, such as an electroporation method (*Methods. Enzymol.*, 194, 182-187 (1990)), a spheroplast method (*Proc. Natl. Acad*. *Sci*., *USA*, 84, 1929 (1978)), a lithium acetate method (*J*. *Bacteriol.*, 153, 163 (1983)) and the like, can be used.

When animal cells are used as the host, pAGE107 (Japanese Published Unexamined Patent Application No. 22979/91; *Cytotechnology*, 3, 133 (1990)), pAGE103 (*J. Biochem.,* 101, 1307 (1987)) and the like, may be used as the expression vector.

Any promoter can be used, so long as it functions in an animal cell. Examples include the promoter of IE (immediate early) gene of cytomegalovirus (CMV), the SV40 promoter, the metallothionein promoter and the like. Also, the enhancer of the IE gene of human CMV may be used together with the promoter.

As the method for the introducing the recombinant vector into an animal cell, any one of the known methods for introducing DNA into an animal cell, such as an electroporation method (*Cytotechnology*, 3, 133 (1990)), a calcium phosphate method (Japanese Published Unexamined Patent Application No. 227075/90), a lipofection method (*Proc. Natl*. *Acad. Sci., USA*, 84, 7413 (1987)) and the like, can be used.

When insect cells are used as the host, the protein can be expressed by the known method described, for example, in *Current Protocols in Molecular Biology*, Supplement 1-34 and *Baculovirus Expression Vectors*, A laboratory manual. That is, a protein-expressing insect cell is obtained by simultaneously introducing the recombinant gene-introducing vector and baculovirus described below to obtain a recombinant virus in the insect cell culture supernatant and then infecting the insect cell with the thus obtained recombinant virus.

The gene introducing vector includes pVL1392, pVL1393, pBlueBacIII (all manufactured by Invitrogen) and the like.

The baculovirus includes *Autographa californica* nuclear polyhedrosis virus with which insects of the family *Barathra* are infected.

The method for simultaneously introducing the above recombinant gene introducing vector and the above baculovirus into an insect cell for the preparation of the recombinant virus includes a calcium phosphate method (Japanese Published Unexamined Patent Application No. 227075/90), a lipofection method (*Proc. Natl. Acad. Sci., USA*, 84, 7413 (1987)) and the like.

Alternatively, the protein can be produced by preparing a recombinant baculovirus making use of BaculoGold Starter Kit manufactured by Pharmigen or the like and then infecting the above insect cell such as Sf9, Sf21, High Five or the like with the recombinant virus (*Bio/Technology*, 6, 47 (1988)).

As the gene expression method, secretion production, fusion protein expression and the like have been developed in addition to the direct expression, and any of such methods can be used. For example, it can be carried out in accordance with the method described in (*Molecular Cloning*, 2nd edition, Cold Spring Harbor Lab. Press, New York (1989); hereinafter referred to as "*Molecular Cloning*, 2nd edition").

The full length or a partial fragment of the human VEGF receptor Flt-1 can be produced as such or as a fusion protein thereof, by culturing the thus obtained transformant in a medium to produce and accumulate the full length or a partial fragment of the human VEGF receptor Flt-1 in the resulting culture, and then recovering the product from the culture.

The above method for culturing the transformant in a medium is carried out in accordance with a usual method which is used in culturing the host cell.

As the medium for use in culturing the transformant obtained using a microorganism such as *Escherichia coli*, yeast or the like as the host, any of a natural medium and a synthetic medium can be used, so long as it contains a carbon source, a nitrogen source, an inorganic salt and the like which can be assimilated by the microorganism and the transformant is efficiently cultured therein (*Molecular Cloning*, 2nd edition). Culturing is carried out generally under aerobic conditions such as shaking culture, submerged agitation aeration culture or the like at 15 to 40°C for 16 to 96 hours. During culturing, the pH is maintained at 3.0 to 9.0. The pH is adjusted using an inorganic or organic acid, an alkali solution, urea, calcium carbonate, ammonia and the like. Antibiotics such as ampicillin, tetracycline and the like can be optionally added to the medium during culturing.

As the medium for use in culturing a transformant obtained using animal cells as the host, usually used RPMI 1640 medium, Eagle's MEM medium or any one of these media further supplemented with fetal calf serum can be used. Culturing is carried out generally at 35 to 37°C for 3 to 7 days in the presence of 5% CO₂, and antibiotics such as kanamycin, penicillin and the like can be optionally added to the medium during culturing.

As the medium for use in culturing a transformant obtained using insect cells as the host, usually used TNM-FH medium (manufactured by Pharmingen), Sf900IISFM (manufactured by Life Technologies), ExCell400 or ExCell405 (both manufactured by JRH Biosciences) or the like can be used. Culturing is carried out generally at 25 to 30°C for 1 to 4 days, and antibiotics such as gentamicin and the like can be optionally added to the medium during culturing.

Also, if it is possible in culturing animal cells and insect cells, it is preferred to use a serum-free medium in order to facilitate purification of the full length or a partial fragment of the human VEGF receptor Flt-1 as such or as a fusion protein.

When the complete length or a partial fragment of the human VEGF receptor Flt-1 is accumulated inside the host cells as such or as a fusion protein, the cells after completion of culturing are centrifuged, suspended in an aqueous buffer and disrupted using an ultrasonic oscillator method, a French press method or the like, and then the protein is recovered from the supernatant after centrifugation.

Also, when an insoluble material is formed inside the cells, the insoluble material is solubilized using a protein denaturing agent and then a higher-order structure of the protein can be formed by diluting or dialyzing the protein in or against a solution which contains no protein denaturing agent or contains the protein denaturing agent in such a low concentration that the protein is not denatured.

When the full length or a partial fragment of the human VEGF receptor Flt-1 is secreted outside the cells as such or as a fusion protein, the expressed protein can be recovered from the culture supernatant.

The isolation and purification can be carried out by employing separation means, such as solvent extraction, fractional precipitation with organic solvents, salting out, dialysis, centrifugation, ultrafiltration, ion exchange chromatography, gel filtration chromatography, hydrophobic chromatography, affinity chromatography, reverse phase chromatography, crystallization, electrophoresis and the like, alone or in combination.

The polypeptide having a partial sequence can also be produced by a chemical synthesis method such as a Fmoc method (fluorenylmethoxycarbonyl method), a tBoc method (t-butyloxycarbonyl method) or the like. It can also be produced using a peptide synthesizer available from Sowa Boeki (manufactured by Advanced chemTech), Perkin-Elmer Japan (manufactured by Perkin-Elmer, USA), Aroka (manufactured by Protein Technology Instrument, USA), Krabow (manufactured by Synthecell-Vega, USA), Japan PerSeptive Ltd. (manufactured by PerSeptive, USA), Shimadzu Corp. or like.

### (2) Immunization of animal and preparation of antibody-producing cell

An animal is immunized using the thus obtained protein as the antigen. As the immunization method, the antigen may be administered as such to an animal subcutaneously, intravenously or intraperitoneally, but it is preferred to administer the antigen by binding a carrier protein having a high antigenicity thereto or together with an appropriate adjuvant.

Examples of the carrier protein include Fissurellidae hemocyanin, keyhole limpet hemocyanin, bovine serum albumin, bovine thyroglobulin and the like. Examples of the adjuvant include complete Freund's adjuvant, a combination of aluminum hydroxide with pertussis vaccine and the like.

The animal to be immunized includes non-human mammals such as rabbit, goat, mouse, rat, hamster and the like.

After the first administration, the antigen may be administered every 1 to 2 weeks for 3 to 10 times. A dose of the antigen is preferably from 50 to 100 µg per animal. A blood sample is collected from the fundus of the eye or caudal vein of the immunized animal 3 to 7 days after each administration and tested, for example, by an enzyme immunoassay ((Enzyme-linked Immunosorbent Assay (ELISA), published by Igaku Shoin, (1976)) on the reactivity of the serum with the antigen.

A non-human animal showing a sufficient antibody titer in its serum is used as the supply source of antibody-producing cells.

Three to seven days after final administration of the antigen, lymphocytes are collected from the immunized animal in accordance with the known method (*Antibodies, A Laboratory Manual*) to fuse the lymphocytes with myeloma cells.

The polyclonal antibody can be prepared by separating and purifying the serum.

The monoclonal antibody can be obtained by preparing a hybridoma through fusion of an antibody-producing cell with a myeloma cells derived from a non-human mammal, culturing the hybridoma, or administering the cells to a mammal to cause ascites tumor, and then separating and purifying the culture or ascitic fluid.

The antibody-producing cell is collected from the spleen cells, lymph node, peripheral blood or the like of the antigen-administered non-human mammal.

### (3) Preparation of myeloma cell

As the myeloma cell, any myeloma cell capable of growing *in vitro* can be used. Examples include established cells obtained from mouse, such as 8-azaguanine-resistant mouse (BALB/c) myeloma cell lines P3-X63Ag8-U1 (P3-U1) (*Eur. J. Immunol*, 6, 511 (1976)), SP2/O-Ag14 (SP-2) (*Nature*, 276, 269 (1978)), P3-X63-Ag8653 (653) (J. *Immunol*., 123, 1548 (1979)), P3-X63-Ag8 (X63) (*Nature*, 256, 495 (1975)) and the like. These cell lines are cultured and subcultured in accordance with the known method (*Antibodies*, *A Laboratory Manual*) and 2×10⁷ or more of the cells are secured until cell fusion.

### (4) Cell fusion and selection of monoclonal antibody

The antibody-producing cells and myeloma cells obtained in the above are washed, mixed with a cell aggregating medium such as polyethylene glycol-1000 (PEG-1000) or the like to fuse the cells and then suspended in a medium. The cells are washed with MEM medium, PBS (1.83 g of disodium hydrogen phosphate, 0.21 g of potassium dihydrogen phosphate, 7.65 g of sodium chloride, 1 liter of distilled water, pH 7.2) or the like. In order to obtain the fused cells of interest selectively, HAT medium {normal medium (a medium prepared by adding glutamine (1.5 mM), 2-mercaptoethanol (5×10⁻⁵ M), gentamicin (10 µg/ml) and fetal calf serum (FCS) (10%, manufactured by CSL) to RPMI-1640 medium) further supplemented with hypoxanthine (10⁻⁴ M), thymidine (1.5×10⁻⁵ M) and aminopterin (4×10⁻⁷ M)} is used as the medium for suspending the fused cells.

After culturing, a portion of the culture supernatant is sampled and tested by an enzyme immunoassay to select a sample which reacts with the antigen protein but does not react with non-antigen proteins. Thereafter, cloning is carried out by limiting dilution analysis, and a hybridoma which shows a stable and high antibody titer by the following enzyme immunoassay is selected as the hybridoma capable of producing the monoclonal antibody.

### Enzyme immunoassay:

The antigen protein, a cell expressing the antigen protein or the like is coated on a plate and allowed to react with a hybridoma culture supernatant or a purified antibody obtained by the above method as a first antibody.

After the first antibody reaction, the plate is washed and a second antibody is added.

The second antibody is an antibody which can recognize the first antibody and is labeled with biotin, an enzyme, a chemiluminescent substance, a radioactive compound or the like. Specifically, when mouse is used in the preparation of hybridoma, an antibody which can recognize mouse immunoglobulin is used as the second antibody.

After the reaction, a reaction suitable for the second antibody-labeled substance is carried out to select a hybridoma capable of producing a monoclonal antibody which specifically reacts with the antibody.

Specific examples of the anti-Flt-1 monoclonal antibody include monoclonal antibody KM1732 belonging to the IgG1 subclass which is produced by hybridoma KM1732 (FERM BP-5698), monoclonal antibody KM1730 belonging to the IgG2b subclass which is produced by hybridoma KM1730 (FERM BP-5697), monoclonal antibody KM1731 belonging to the IgG2a subclass which is produced by hybridoma KM1731 (FERM BP-5718), monoclonal antibody KM1748 belonging to the IgG2b subclass which is produced by hybridoma KM1748 (FERM BP-5699), monoclonal antibody KM1750 which belonging to the IgG2b subclass which is produced by hybridoma KM1750 (FERM BP-5700) described in WO 98/22616 and the like.

### (5) Preparation of monoclonal antibody

The monoclonal antibody can be prepared by separating and purifying it from a culture obtained by culturing a hybridoma cell or from an ascitic fluid of an 8- to 10-week-old mouse or nude mouse in which ascites tumor was induced by treating the animal with pristane (by intraperitoneal administration of 0.5 ml of 2,6,10,14-tetramethylpentadecane (pristane), followed by feeding for 2 weeks) and then intraperitoneally administering a monoclonal antibody-producing hybridoma cell.

The method for separating and purifying the monoclonal antibody includes centrifugation, salting out with 40 to 50% saturated ammonium sulfate, caprylic acid precipitation and chromatography using a DEAE-Sepharose column, an anion exchange column, a protein A or G column, a gel filtration column or the like, which may be used alone or in combination. By this method, a purified monoclonal antibody can be obtained by recovering an IgG or IgM fraction.

The subclass of the purified monoclonal antibody can be determined using a monoclonal antibody typing kit or the like. The amount of the protein can be calculated by the Lowry method or based on the absorbance at 280 nm.

The subclass of the antibody is an isotype in a class. Examples in mouse include IgG1, IgG2a, IgG2b and IgG3, and examples in human include IgG1, IgG2, IgG3 and IgG4. Particularly since mouse IgG1 and IgG2a and human IgG1-types have complement-dependent cytotoxic activity (hereinafter "CDC activity") and antibody-dependent cellular cytotoxic activity (hereinafter "ADCC activity"), they are useful in applying to medical treatments.

### 2. Production method of recombinant antibody (1)

Production method of anti-human VEGF receptor Flt-1 humanized antibody:

### (1) Construction of humanized antibody expression vector

A vector for humanized antibody expression necessary for producing a humanized antibody from an antibody of an animal other than human is constructed. The humanized antibody expression vector is an expression vector for animal cell into which genes encoding the human antibody C regions CH and CL have been incorporated, which is constructed by inserting respective genes encoding CH and CL of a human antibody into an expression vector for animal cell.

As the human antibody C region, the C region of an optional human antibody can be used, such as Cγ1 and Cγ4 in human antibody H chain and Cκ in human antibody L chain. As the gene encoding a human antibody C region, a chromosomal DNA composed of exon and intron can be used, and a cDNA can also be used. As the expression vector for animal cell, any vector can be used, so long as a gene encoding a human antibody C region can be incorporated and expressed.

Examples include pAGE107 (*Cytotechnology*, 3, 133 (1990)), pAGE103 (*J. Biochem.*, 101, 1307 (1987)), pHSG274 *(Gene,* 27, 223 (1984)), pKCR (*Proc. Natl. Acad. Sci. USA*, 78, 1527 (1981)), pSG1βd2-4 (*Cytotechnology*, 4, 173 (1990)) and the like. The promoter and enhancer used in the expression vector for animal cell include, SV40 early promoter and enhancer (*J. Biochem*., 101, 1307 (1987)), Moloney mouse leukemia virus LTR promoter and enhancer (*Biochem*. *Biophys. Res. Comun.*, 149, 960 (1987)), immunoglobulin H chain promoter (*Cell*, 41, 479 (1985)) and enhancer (*Cell*, 33, 717 (1983)) and the like.

As the humanized antibody expression vector, any of a type in which the antibody H chain and L chain are present on separate vectors and a type in which they are present on the same vector (tandem-type) can be used, but a tandem-type humanized antibody expression vector is preferred from the viewpoint that the humanized antibody expression vector can be easily constructed, its introduction into an animal cell is easy, and expression amounts of the antibody H chain and L chain in the animal cell can be balanced (*J. Immunol*. *Methods*, 167, 271 (1994)).

### (2) Preparation of cDNA encoding VH and VL of antibody of animal other than human

cDNA encoding the VH and VL of an antibody of an animal other than human, such as a mouse anti-human VEGF receptor Flt-1 monoclonal antibody, is obtained, for example, as follows.

cDNA is synthesized by extracting mRNA from an anti-human VEGF receptor Flt-1 monoclonal antibody-producing cell, such as a mouse anti-human VEGF receptor Flt-1 antibody-producing hybridoma or the like. The thus synthesized cDNA is inserted into a phage vector, plasmid vector or the like to prepare a cDNA library. A recombinant phage or recombinant plasmid containing cDNA encoding VH and a recombinant phage or recombinant plasmid containing cDNA encoding VL are isolated from the library using part of C region and part of V region, respectively, of an antibody of an animal other than human, such as a mouse antibody, as the probe. Full nucleotide sequences of the antibody VH and VL of interest on the recombinant phage or recombinant plasmid are determined, and full amino acid sequences of the VH and VL are deduced from the nucleotide sequences.

### (3) Construction of human chimeric antibody expression vector

A human chimeric antibody expression vector can be constructed by inserting cDNA encoding VH and VL of an antibody of an animal other than human into the upstream of a gene encoding CH and CL of a human antibody on the humanized antibody expression vector constructed in the above 2(1). For example, a human chimeric antibody expression vector can be produced by arranging a restriction enzyme recognizing sequence for use in the cloning of cDNA encoding VH and VL of an antibody of an animal other than human, in advance upstream of a gene encoding CH and CL of a chimeric antibody expression vector, and inserting cDNA encoding V regions of an antibody of an animal other than human into this cloning site via a synthetic DNA described below. The synthetic DNA contains a 3'-terminal nucleotide sequence of the V region of an antibody of an animal other than human and a 5'-terminal nucleotide sequence of the C region of a human antibody and can be produced using a DNA synthesizer in such a manner that it has appropriate restriction enzyme sites on both termini.

### (4) Identification of CDR sequence of antibody of animal other than human

The VH and VL which form the antigen binding region of an antibody are composed of four frame work regions (hereinafter referred to as "FR region") whose sequences are relatively preserved and three complementarity determining regions (CDR) which connects them and is rich in sequence changes (*Sequences of Proteins of Immunological Interest*, US Dep. Health and Human Services, 1991: hereinafter referred to as *"Sequences of Proteins of Immunological Interest"*). Each CDR amino acid sequence (CDR sequence) can be identified by comparing it with amino acid sequences of V regions of known antibodies (*Sequences of Proteins of Immunological Interest*).

### (5) Construction of cDNA encoding V regions of human CDR-grafted antibody

The cDNA encoding the VH and VL of human CDR-grafted antibody can be obtained as follows.

First, the amino acid sequence of FR of the V region of a human antibody for grafting CDR of the V region of an antibody of an animal other than human is selected for both VH and VL. As the amino acid sequence of FR of the V region of a human antibody, any amino acid sequence can be used, so long as it is an amino acid sequence of FR of the V region derived from a human antibody.

For example, amino acid sequences of FR of V regions of human antibodies registered at Protein Data Bank and an amino acid sequence common in each subgroup of FR of V regions of human antibodies (*Sequences of Proteins of Immunological Interest*) can be cited, but in order to create a human CDR-grafted antibody having sufficient activity, it is preferred that it has a high homology, preferably a homology of 65% or more, with the intended amino acid sequence of V regions of an antibody of an animal other than human. Next, a DNA sequence encoding the thus selected amino acid sequence of FR of the V region of human antibody is linked to a DNA sequence encoding the intended amino acid sequence of CDR of the V region of an antibody of an animal other than human, and DNA sequences encoding amino acid sequences of VH and VL, respectively are designed. In order to obtain the DNA sequences designed for constructing a CDR-grafted antibody variable region gene, several synthetic DNA fragments are designed for each chain in such a manner that the complete DNA sequence is covered, and a polymerase chain reaction (hereinafter referred to as "PCR") is carried out using these fragments. Based on the reaction efficiency in the PCR and the length of DNA which can be synthesized, preferably 6 synthetic DNA fragments are designed for each chain. After the reaction, the thus amplified fragments are subcloned in an appropriate vector and their nucleotide sequences are determined to thereby obtain a plasmid containing cDNA encoding the amino acid sequence of the V region of each chain of the human CDR-grafted antibody of the interest. Alternatively, cDNA encoding the amino acid sequence of the V region of each chain of the human CDR-grafted antibody of the interest can also be constructed by synthesizing complete sense and antisense sequences using a synthetic DNA fragment of about 100 bases, and annealing and linking them.

### (6) Modification of amino acid sequence of V region of human CDR-grafted antibody

It is known that when only the CDR of the V region of an antibody of an animal other than human of interest is simply grafted between FR's of the V region of a human antibody, the activity of the human CDR-grafted antibody is reduced in comparison with the activity of the original antibody of an animal other than human (*BIO/TECHNOLOGY*, 9, 266 (1991)). Accordingly, attempts have been made to increase the activity by a method in which, among amino acid sequences of FR's of V regions of a human antibody, an amino acid residue directly relating to the binding to the antigen, an amino acid residue interacting with an amino acid residue of CDR or an amino acid residue having a possibility of relating to the maintenance of three-dimensional structure of the antibody or the like is changed to an amino acid residue found in the original antibody of an animal other than human. In order to identify these amino acid residues efficiently, construction and analysis of the three-dimensional structure of antibodies have been carried out using an X-ray crystal analysis, computer modeling or the like. However, since a method for producing a human CDR-grafted antibody applicable to every antibody has not yet been established, it is necessary under the present situation to carry out various trial and error investigations on individual antibodies.

The selected amino acid sequence of the FR of V region of a human antibody can be modified by carrying out the PCR described in the above 2(5) using various mutation-introduced primers. The amplified fragments after the PCR are subcloned in an appropriate vector and their nucleotide sequences are determined to obtain a vector containing cDNA introduced with the mutation of interest (hereinafter referred to as "amino acid sequence-modified vector").

Also, in the modification of an amino acid sequence within a narrow range, it can be carried out by a PCR mutation introducing method using mutation-introduced primers consisting of 20 to 35 nucleotides. Specifically, a sense mutated primer and antisense mutated primer consisting of 20 to 35 nucleotides containing a DNA sequence encoding the amino acid residues after modification are synthesized, and two step PCR is carried out using a plasmid containing cDNA encoding the V region amino acid sequence to be modified as the template. The finally amplified fragments are subcloned in an appropriate vector and their nucleotide sequences are determined to obtain an amino acid sequence-modified vector containing cDNA introduced with the mutation of interest.

### (7) Construction of human CDR-grafted antibody expression vector

A human CDR-grafted antibody expression vector can be constructed by inserting the cDNA encoding human CDR-grafted antibody VH and VL obtained in the above 2(5) and 2(6) into the upstream of the gene encoding human antibody CH and CL in the humanized antibody expression vector of the above 2(1). For example, appropriate restriction enzyme recognizing sequences are introduced into the of 5'-end and 3'-end termini of the synthetic DNA in PCR for constructing cDNA encoding amino acid sequences of the VH and VL of human CDR-grafted antibody so that they can be inserted into the upstream of the gene encoding the desired human antibody C region in such a manner that they can be expressed in a suitable form.

### (8) Transient expression and activity evaluation of humanized antibody

In order to evaluate the activity of various humanized antibodies efficiently, their activities can be measured by carrying out transient expression of humanized antibodies (*Methods in Nucleic Acids Res.*, CRC Press, p. 283, 1991), by introducing the human chimeric antibody expression vector of the above 2(3) and the human CDR-grafted antibody expression vector of the above 2(7) or a modified vector thereof into COS-7 cell (ATCC CRL 1651).

The method for introducing an expression vector into COS-7 cell includes a DEAE-dextran method (*Methods in Nucleic Acids Res.,* CRC Press, p. 283, 1991), a lipofection method (*Proc. Natl. Acad. Sci.*, 84, 7413 (1987)) and the like.

After introduction of the vector, the activity of the humanized antibody in a culture supernatant can be measured by the enzyme immunoassay (ELISA) described in the above 1(4) or the like.

### (9) Stable expression and activity evaluation of humanized antibody

A transformant capable of producing a humanized antibody stably can be obtained by introducing the human chimeric antibody expression vector of the above 2(3) and the human CDR-grafted antibody expression vector of the above 2(7) into an appropriate host cell.

The method for introducing an expression vector into a host cell includes an electroporation method (Japanese Published Unexamined Patent Application No. 257891/90, *Cytotechnology*, 3, 133 (1990)) and the like.

As the host cell into which a humanized antibody expression vector is introduced, any cell can be used, so long as it is a host cell which can express the humanized antibody. Examples include mouse SP2/0-Ag14 cell (ATCC CRL 1581), mouse P3X63-Ag8.653 cell (ATCC CRL 1580), CHO cell from which dihydrofolate reductase gene (hereinafter referred to as "DHFR gene") has been deleted (*Proc. Natl. Acad. Sci. USA*, 77, 4216 (1980)), rat YB2/3HL.P2.G11.16Ag.20 cell (ATCC CRL 1662, hereinafter referred to as "YB2/0 cell") and the like.

After introduction of the vector, a transformant capable of stably producing the humanized antibody is selected using RPMI1640 medium containing G418 and FCS in accordance with the method disclosed in Japanese Published Unexamined Patent Application No. 257891/90. By culturing the thus obtained transformant in a medium, the humanized antibody can be produced and accumulated in the culture. The activity of the humanized antibody in the culture is measured by the method described in the above 1(4) or the like. Also, the produced amount of the humanized antibody can be increased in accordance with the method disclosed in Japanese Published Unexamined Patent Application No. 257891/90, making use of a DHFR gene amplification system.

The humanized antibody can be purified from a culture supernatant of the transformant using a protein A column (*Antibodies, A Laboratory Manual*, Cold Spring Harbor Laboratory, Chapter 8, 1988; hereinafter referred to as *"Antibodies"*). In addition to this, a purification method generally used for a protein can also be used. For example, its purification can be carried out through the combination of gel filtration, ion exchange chromatography, ultrafiltration and the like. The molecular weight of the H chain, L chain or entire antibody molecule of the purified humanized antibody is measured by polyacrylamide gel electrophoresis (SDS-PAGE) (*Nature*, 227, 680 (1970)), Western blotting method (*Antibodies*, Chapter 12, 1988) or the like.

The reactivity of the purified humanized antibody or the inhibition activity of the humanized antibody against VEGF can be measured by the method described in the above 1(4) or the like.

### 3. Production method of recombinant antibody (2)

### (1) Method for producing antibody fragments Fab, Fab' and F(ab')₂

An antibody fragment is formed by treating the above antibody with an enzyme. The enzyme includes papain, trypsin and the like.

Alternatively, Fab, Fab' or F(ab')₂ can be produced by inserting DNA encoding the Fab, Fab' or F(ab')₂ fragment of the human VEGF receptor Flt-1 antibody into an expression vector for animal cell and introducing the vector into an animal cell to thereby express it.

The thus formed antibody fragment can be purified using the combination of gel filtration, ion exchange or affinity chromatography, ultrafiltration and the like. The molecular weight of the purified Fab, Fab' or F(ab')₂ is measured by polyacrylamide gel electrophoresis (SDS-PAGE) (*Nature*, 227, 680 (1970)), Western blotting (*Antibodies*, Chapter 12, 1988) or the like.

The reactivity of the purified Fab, Fab' or F(ab')₂ or the inhibition activity of the Fab, Fab' or F(ab')₂ against VEGF can be measured by the method described in the above 1(4) or the like.

### (2) Method for producing anti-human VEGF receptor Flt-1 single chain antibody

An expression vector for a single chain antibody of an antibody of an animal other than human or a single chain antibody of a human CDR-grafted antibody can be constructed by inserting the cDNA encoding VH and VL of the antibody of an animal other than human or the human CDR-grafted antibody described in the above 2(2), 2(5) and 2(6) into an expression vector for single chain antibody. As the expression vector for single chain antibody, any vector can be used, so long as the cDNA encoding VH and VL of the antibody of an animal other than human or the human CDR-grafted antibody can be incorporated and expressed.

Examples include pAGE107 (*Cytotechnology*, 3, 133 (1990)), pAGE103 (*J. Biochem*., 101, 1307 (1987)), pHSG274 (*Gene,* 27, 223 (1984)), pKCR (*Proc. Natl. Acad. Sci. USA*, 78, 1527 (1981)), pSG1βd2-4 (*Cytotechnology*, 4, 173 (1990)) and the like. As the host for expressing the single chain antibody, a suitable cell can be selected from *E. coli,* yeast, animal cells and the like, but in that case, it is necessary to select an expression vector suitable for each host. Also, the single chain antibody can be secreted into the outside of the cell, transported into the periplasmic region or retained inside the cell, by inserting cDNA encoding an appropriate signal peptide into the expression vector.

A single chain antibody expression vector into which cDNA encoding the single chain antibody of interest is inserted can be constructed by inserting cDNA encoding a single chain antibody composed of VH-P-VL or VL-P-VH (P is a peptide linker) into the downstream of an appropriate promoter and a signal peptide region of the thus selected expression vector.

The cDNA encoding the single chain antibody can be obtained by linking cDNA encoding VH to cDNA encoding VL using a synthetic DNA fragment encoding a peptide linker having appropriate restriction enzyme recognizing sequences on both termini. It is important to optimize the linker peptide in such a manner that its addition does not obstruct binding of VH and VL to the antgen, and, for example, a peptide shown by Pantoliano *et al.* (*Biochemistry*, 30, 10117 (1991)) or a modified product thereof can be used.

### (3) Method for producing anti-human VEGF receptor Flt-1 disulfide stabilized antibody

A disulfide stabilized antibody can be produced by substituting a DNA sequence corresponding to an amino acid residue at an appropriate position of the cDNA encoding VH and VL of the antibody of an animal other than human or the cDNA encoding VH and VL of the human CDR-grafted antibody with a DNA sequence corresponding to a cysteine residue, followed by expression and purification, and then forming a disulfide bond. The amino acid residue can be substituted with a cysteine residue by the mutation introducing method using the PCR described in the above 2(5).

A disulfide stabilized antibody H chain expression vector and a disulfide stabilized antibody L chain expression vector can be constructed by inserting the thus obtained cDNA encoding the modified VH and modified VL into an appropriate expression vector. As the vector for disulfide stabilized antibody expression, any vector can be used, so long as it can incorporate and express the cDNA encoding the modified VH and modified VL. Example includes pAGE107 (*Cytotechnology*, 3, 133 (1990)), pAGE103 (*J. Biochem*., 101, 1307 (1987)), pHSG274 (*Gene*, 27, 223 (1984)), pKCR (*Proc. Natl. Acad. Sci. USA*, 78, 1527 (1981)), pSG1βd2-4 (*Cytotechnology, 4*, 173 (1990)) and the like. As the host for expressing the disulfide stabilized antibody L chain expression vector and disulfide stabilized antibody H chain expression vector used for forming a disulfide stabilized antibody, an appropriate cell can be selected from *E. coli,* yeast, animal cells and the like, but in that case, it is necessary to select an expression vector suitable for each host. Also, the disulfide stabilized antibody can be secreted into the outside of the cell, transported into the periplasmic region or retained inside the cell, by inserting cDNA encoding an appropriate signal peptide into the expression vector.

### (4) Expression and activity evaluation of various antibodies

A transformant capable of producing the antibody fragment, single chain antibody, disulfide stabilized antibody H chain or disulfide stabilized antibody L chain of interest can be obtained by introducing the antibody fragment expression vector, single chain antibody expression vector, disulfide stabilized antibody H chain expression vector or disulfide stabilized antibody L chain expression vector constructed in the above (1) to (3) into a host cell by an electroporation method (Japanese Published Unexamined Patent Application No. 257891/90, *Cytotechnology*, 3, 133 (1990)) or the like. After introduction of the expression vector, expression of the antibody fragment, single chain antibody, disulfide stabilized antibody H chain or disulfide stabilized antibody L chain contained in a culture supernatant or the like can be confirmed by the method described in the above 1(4) or the like.

The antibody fragment, single chain antibody, disulfide stabilized antibody H chain or disulfide stabilized antibody L chain can be recovered and purified by combining known techniques. For example, when the antibody fragment, single chain antibody, disulfide stabilized antibody H chain or disulfide stabilized antibody L chain is secreted into a culture medium, it can be concentrated by ultrafiltration, and then it can be recovered and purified by various chromatography or gel filtration. Also, when it is transported into a periplasmic region of the host cell, it can be concentrated by ultrafiltration after adding an osmotic pressure shock to the cell, and then it can be recovered and purified by various chromatography or gel filtration. When the antibody fragment, single chain antibody, disulfide stabilized antibody H chain or disulfide stabilized antibody L chain is insoluble and present as granules (inclusion body), centrifugation and washing for dissolving the cell and isolating granules are repeated, it is solubilized, for example with guanidine-hydrochloric acid, and then it can be recovered and purified by various chromatography or gel filtration.

The activity of the purified antibody fragment or single chain antibody can be measured by the method described in the above 1(4) or the like.

The purified disulfide stabilized antibody H chain and disulfide stabilized antibody L chain are mixed and a disulfide bond is formed by a technique for leading to an active structure (refolding procedure, *Molecular Immunology*, 32, 249 (1995)), and then the disulfide stabilized antibody having the activity can be purified by antigen affinity chromatography, ion exchange chromatography or gel filtration. The activity of the disulfide stabilized antibody can be measured by the method described in the above 1(4) or the like.

### 4. Method for producing fusion antibody

A fusion antibody prepared by fusing an antibody or antibody fragment used in the present invention with a radioisotope, a protein, a low molecular weight agent or the like by a chemical or genetic engineering means can also be used as an antibody analogue. A fusion antibody in which an antibody and a toxin protein are chemically fused can be prepared in accordance with, for example, *Anticancer Research*, 11, 2003 (1991); *Nature Medicine*, 3, 350 (1996). A fusion antibody in which an antibody is fused with a protein such as toxin, cytokine or the like by genetic engineering techniques can be prepared in accordance with, for example, *Proc. Natl. Acad. Science, USA,* 93, 974 (1996), *Proc. Natl. Acad. Science, USA,* 93, 7826 (1996). A fusion antibody in which an antibody is chemically fused with a low molecular weight anticancer agent can be prepared in accordance with, for example, *Science,* 261, 212 (1993). A fusion antibody in which an antibody is chemically fused with a radioisotope can be prepared in accordance with, for example, *Antibody Immunoconjugates and* *Radiopharmaceuticals*, 3, 60 (1990); *Anticancer Research*, 11, 2003 (1991).

Since these analogues can accumulate a radioisotope, a protein (a cytokine, a toxin, an enzyme or the like), a low molecular weight agent or the like into the periphery of a target tissue according to the specificity of the antibody molecule, a diagnosis or treatment which is more effective and has less side effects can be expected from them.

### 5. Application method of antibody

Since the above anti-Flt-1 antibody, the antibody fragment or the fusion antibody thereof with other molecule binds to the human VEGF receptor Flt-1 and destroys Flt-1-expressing cells through an antibody effector activity such as ADCC, CDC or the like, they are considered to be useful in the treatment of leukemia and the like.

A medicament comprising the antibody of the present invention can be administered as a therapeutic agent alone, but generally, it is preferred to provide it as a pharmaceutical preparation produced by an optional method well known in the technical field of manufacturing pharmacy, by mixing it with one or more pharmaceutically acceptable carriers.

It is preferred to use a route of administration which is most effective in a treatment. Examples include oral administration and parenteral administration such as buccal, airway, rectal, subcutaneous, intramuscular or intravenous administration. Intravenous administration is preferred in an antibody preparation.

The dosage form includes sprays, capsules, tablets, granules, syrups, emulsions, suppositories, injections, ointments, tapes and the like.

Liquid preparations such as emulsions and syrups can be produced using, as additives, water; saccharides such as sucrose, sorbitol, fructose, etc.; glycols such as polyethylene glycol, propylene glycol, etc.; oils such as sesame oil, olive oil, soybean oil, etc.; antiseptics such as p-hydroxybenzoic acid esters, etc.; flavors such as strawberry flavor, peppermint, etc.; and the like.

Capsules, tablets, powders, granules and the like can be produced using, as additives, fillers such as lactose, glucose, sucrose, mannitol, *etc.*; disintegrating agents such as starch, sodium alginate, *etc.*; lubricants such as magnesium stearate, talc, etc.; binders such as polyvinyl alcohol, hydroxypropylcellulose, gelatin *etc.*; surfactants such as fatty acid ester, *etc.*; plasticizers such as glycerine, *etc*.; and the like.

Examples of the pharmaceutical preparation suitable for parenteral administration include injections, suppositories, sprays and the like.

The injections are prepared using a carrier such as a salt solution, a glucose solution or a mixture thereof or the like.

The suppositories are prepared using a carrier such as cacao butter, hydrogenated fat, carboxylic acid or the like.

Also, sprays are prepared using the compound as such or using a carrier or the like which does not stimulate the buccal or airway mucous membrane of the patient and can facilitate absorption of the compound by dispersing it as fine particles.

Examples of the carrier include lactose, glycerol and the like. It is possible to produce pharmaceutical preparations such as aerosols, dry powders and the like. Depending on the properties of the antibody and the carrier to be used, in addition, the components exemplified as additives for oral preparations can also be added to these parenteral preparations.

Although the clinical dose or the frequency of administration varies depending on the intended therapeutic effect, administration method, treating period, age, body weight and the like, it is usually from 10 µg/kg to 8 mg/kg per day per adult.

Since the monoclonal antibody for the VEGF receptor Flt-1 shown by the present invention reacts with a leukemia cell at a high frequency, it can be used as a diagnostic agent or therapeutic agent for leukemia.

Also, the method for examining an antitumor effect of the antibody used in the present invention on a leukemia cell includes a complement-dependent cytotoxic activity (CDC activity) measuring method, an antibody-dependent cellular cytotoxic activity (ADCC activity) measuring method and the like as *in vitro* tests, and an antitumor experiment using a tumor system in experimental animal (e.g., mouse, etc.) and the like as *in vivo* tests.

Antitumor experiments such as CDC activity, ADCC activity and the like can be carried out in accordance with the methods described in *Cancer Immunology Immunotherapy,* 36, 373 (1993), *Cancer Research*, 54, 1511 (1994) and the like.

### 6. Method for diagnosing leukemia

The leukemia diagnosing method includes a method in which human VEGF receptor Flt-1 existing in the cells or tissues of a person to be tested is immunologically detected or determined.

The method for immunologically detecting a leukemia cell using an antibody for the VEGF receptor Flt-1, includes a fluorescent antibody, an enzyme-linked immunosorbent assay (ELISA), a radioimmunoassay (RIA), an immunohistochemical staining method (ABC method, CSA method or the like) such as immunological tissue staining, immunological cell staining or the like, Western blotting, an immunoprecipitation method, the above enzyme immunoassay, sandwich ELISA (*Monoclonal Antibody Experimentation Manual* (Kodan-sha Scientific, 1987), *Second Series Biochemical Experimentation Course 5, Immunobiochemical Method* (Tokyo Kagaku Dojin, 1986)) and the like.

The fluorescent antibody method can be carried out using the method described in *Monoclonal Antibodies*: Principles and practice, Third edition (Academic Press, 1996), *Monoclonal Antibody Experimentation Manual* (Kodan-sha Scientific, 1987) or the like. Specifically, this is a method in which a separated cell or tissue or the like is allowed to react with the monoclonal antibody of the present invention and then with an anti-immunoglobulin antibody or binding fragment labeled with a fluorescent material such as fluorescein isothiocyanate (FITC), phycoerythrin or the like, and then the fluorescence dye is measured using a flow cytometer.

The enzyme-linked immunosorbent assay (ELISA) is a method in which a cell or disintegrated solution thereof, a tissue or disintegrated solution thereof, a cell culture supernatant, a serum, a pleural effusion, an ascites, an eye fluid or the like is allowed to react with the antibody and then with an anti-immunoglobulin antibody or binding fragment treated with an enzyme label such as peroxidase, biotin or the like, and then the colored dye is measured using a spectrophotometer.

The radioimmunoassay (RIA) is a method in which a cell or disintegrated solution thereof, a tissue or disintegrated solution thereof, separated sample such as a cell culture supernatant, a serum, a pleural effusion, an ascites, an eye fluid or the like is allowed to react with the antibody and then with an anti-immunoglobulin antibody or binding fragment treated with a radioactive ray label, and then the radioactivity is measured using a scintillation counter or the like.

The immunological tissue staining or immunological cell staining is a method in which a separated cell or tissue or the like is allowed to react with the antibody and then with an anti-immunoglobulin antibody or binding fragment labeled with a fluorescent material such as fluorescein isothiocyanate (FITC) or the like or an enzyme label such as peroxidase, biotin or the like, and then the sample is observed using a microscope.

The Western blotting is a method in which a cell extract of a Flt-1-expressed microorganism, animal cell or insect cell is fractionated by SDS-polyacrylamide gel electrophoresis [*Antibodies-A Laboratory Manual*, Cold Spring Harbor Laboratory, 1988] and then blotted on a nitrocellulose membrane, the membrane is allowed to react with the monoclonal antibody of the present invention and then with an anti-mouse IgG antibody or binding fragment labeled with a fluorescent material such as FITC or the like or an enzyme label such as peroxidase, biotin or the like, and then the reaction result is confirmed.

The immunoprecipitation is a method in which a cell extract of a Flt-1-expressed microorganism, animal cell or insect cell is allowed to react with the monoclonal antibody of the present invention and then mixed with a carrier capable of specifically binding to the immunoglobulin (e.g., protein G-Sepharose or the like) to thereby precipitate the antigen-antibody complex.

The sandwich ELISA is a method in which, among two monoclonal antibodies of the present invention having different antigen recognition sites, one of the monoclonal antibodies is adsorbed to a plate in advance, and another monoclonal antibody is labeled with a fluorescent material such as FITC or the like or an enzyme such as peroxidase, biotin or the like. The antibody-adsorbed plate is allowed to react with a cell extract of a Flt-1-expressed microorganism, animal cell or insect cell and then with the labeled monoclonal antibody to carry out the reaction corresponding to the label.

### BRIEF DESCRIPTION OF THE DRAWING

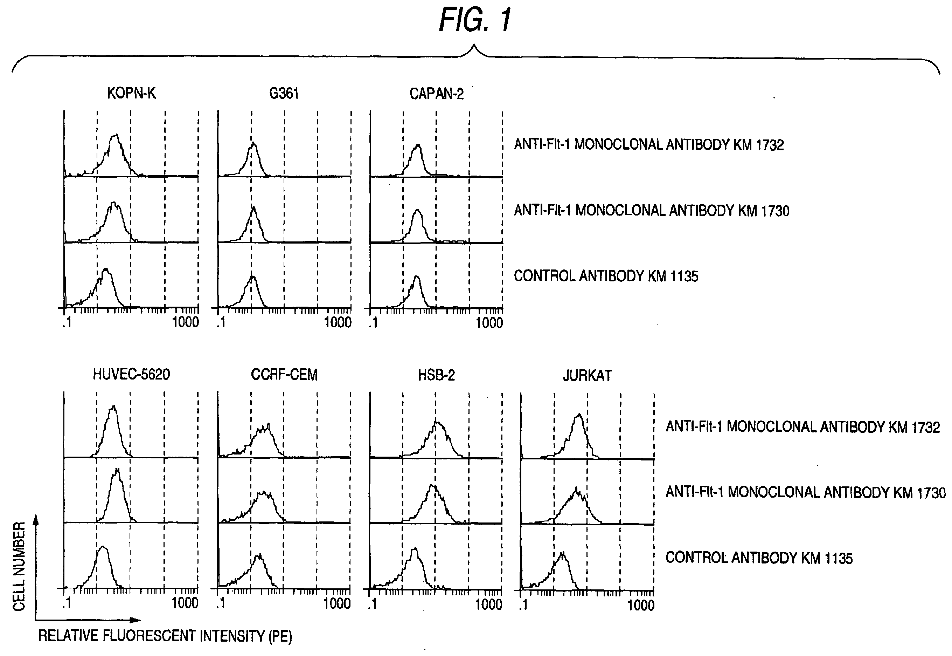

Fig. 1 is a graph showing results of the flow cytometry analysis of the reactivity of anti-human VEGF receptor Flt-1 monoclonal antibodies KM1730 and KM1732 with human cell lines KOPN-K, G361, CAPAN-1, HUVEC, HSB-2 and Jurkat.

### BEST MODE FOR CARRYING OUT THE INVENTION

### Example 1

Production of anti-human VEGF receptor Flt-1 monoclonal antibody:

Each of hybridoma KM1732 (FERM BP-5698), hybridoma KM1730 (FERM BP-5697), hybridoma KM1731 (FERM BP-5718), hybridoma KM1748 (FERM BP-5699)and KM1750 (FERM BP-5700) were intraperitoneally injected to 8-week-age pristane-treated female nude mice (Balb/c) at a dose of 5×10⁶ to 20×10⁶ cells/animal. The hybridomas caused ascites tumor in 10 to 21 days. The ascitic fluid was collected from each ascitic fluid-carrying mouse (1 to 8 ml/animal), centrifuged (3,000 rpm, 5 minutes) for removing solid matter and then purified by a caprylic acid precipitation method (*Antibodies-A Laboratory Manual*) to be used as purified monoclonal antibodies.

The antibody class of these monoclonal antibodies was determined by carrying out an enzyme immunoassay using a subclass typing kit (manufactured by zymed). As a result, KM1732 was a monoclonal antibody belonging to IgG1 subclass, and KM1730 belonging to IgG1 subclass, KM1731 belonging to IgG2a subclass, KM1748 belonging to IgG2b subclass and KM1750 belonging to IgG2b subclass.

### Example 2

Confirmation of reactivity of anti-human Flt-1 monoclonal antibody with human cancer cell line:

The following human cancer cell lines were investigated: pulmonary small cell carcinoma SBC-3 (*Cancer Research*, 54, 1511 (1994)) and SBC-5 (*Cancer Research,* 54, 1511 (1994)), pulmonary squamous cell carcinoma Calu-1 (*Cancer Research*, 54, 1511 (1994)) and PC-10 (*Cancer Research*, 54, 1511 (1994)), pulmonary adenocarcinoma PC-7 (*Cancer Research*, 54, 1511 (1994)) and HLC-1 (*Cancer Research*, 54, 1511 (1994)), large bowel cancer Colo205 (ATCC CCL-222), uterine cancer Hela (ATCC CCL-2), ovarian cancer MCAS (Human Science Resource Foundation JCRB 0240) and OVCAR-3 (purchased from Dainippon Pharmaceutical), gastric cancer (*Cancer Research*, 46, 4438 (1986)) and KatoIII (*Cancer Research*, 46, 4438 (1986)), breast cancer R27 (*Anticancer Research*, 12, 1121 (1992)) and MCF7 (ATCC HTB-22), cancer of pancreas HPAF-2 (ATCC CRL-1997), BXPC-3 (ATCC CRL-1687), Capan-1 (ATCC HTB-79) and Capan-2 (ATCC HTB-80), melanoma G361 (ATCC CRL-1424) and Sk-Mel-28 (ATCC HTB-72), neuroblastoma NAGAI (*Cancer Research*, 54, 1511 (1994)) and YT-nu (*Cancer Research*, 54, 1511 (1994)), glioblastoma A172 (ATCC CRL-1620) and T98G (ATCC CRL-1690), and leukemia Jurkat (ATCC TIB-152), U-937 (ATCC CRL-1593), HL-60 (ATCC CRL-240), HSB-2 (ATCC CCL-120.1), TALL-1 (*Cancer Research*, 54, 1511 (1994)), KOPN-K (*Cancer Research*, 54, 1511 (1994)), NALL-1 (*Cancer Research*, 54, 1511 (1994)), HEL92.1.7 (ATCC TIB-180), HPB-ALL (*Cancer Research*, 54, 1511 (1994)), K562 (ATCC CCL-243), CCRF-CEM (ATCC CCL-119) and CCRF-SB (ATCC CCL-120). Also, human umbilical vein endothelial cell (HUVEC) (purchased from Clonetics) was used as a normal cell.

The reactivity of the anti-human VEGF receptor Flt-1 monoclonal antibody obtained in Example 1 with human cancer cell lines was confirmed in accordance with the following procedure using an immunological cell staining method.

Anti-human VEGF receptor Flt-1 monoclonal antibodies KM1730 and KM1732 and, as a control antibody, a mouse monoclonal antibody KM1135 (WO 96/05230) belonging to mouse IgG1 class which does not react with the human VEGF receptor Flt-1 but react with a human MxA protein were labeled with biotin in the usual way.

Each of the cell lines was suspended in a buffer for immunological cell staining (PBS containing 1% BSA, 0.02% EDTA and 0.05% sodium azide) to a density of 2×10⁵ cells per 100 µl and dispensed into a round bottom 96 well plate. After centrifugation at 4°C and at 350 × g for 1 minute, the supernatant was removed and 50 µl (10 µg/ml) of the biotin-labeled KM1730 antibody, biotin-labeled KM1732 antibody or biotin-labeled KM1135 control antibody was added to each well to carry out a reaction at 4°C for 30 minutes. After the reaction, 200 µl of the buffer for immunological cell staining was added to each well which was subsequently centrifuged at 4°C and at 350 × g for 1 minute, and then the supernatant was discarded and the cells were washed. After further carrying out this washing step twice, 20 µl of the buffer for immunological cell staining containing Avidin-PE (Streptoavidin-R-Phycoerythrin) (manufactured by Gibco) in a concentration of 5 µg/ml was added to carry out a reaction at 4°C for 30 minutes. After the reaction, the above washing step was repeated three times and then the analysis was carried out using a flow cytometer (manufactured by Coulter).

The results are shown in Fig. 1 and Table 1.

**Table 1**

| Target cell | Reactivity | |
|---|---|---|
| | Anti-Flt-1 monoclonal antibody | |
| | KM1730 | KM1732 |
| Pulmonary small cell carcinoma | | |
| SBC-3 | - | - |
| SBC-5 | - | - |

| Pulmonary squamous cell carcinoma | | |
|---|---|---|
| Calu-1 | - | - |
| PC-10 | - | - |

| Pulmonary adenocarcinoma | | |
|---|---|---|
| PC-7 | - | - |
| HLC-1 | - | - |

| Large bowel cancer | | |
|---|---|---|
| Colo205 | - | - |

| Uterine cancer | | |
|---|---|---|
| Hela | - | - |

| Ovarian cancer | | |
|---|---|---|
| MCAS | - | - |
| OVCAR-3 | - | - |

| Gastric cancer | | |
|---|---|---|
| MKN-1 | - | - |
| KatoIII | - | - |

| Breast cancer | | |
|---|---|---|
| R27 | - | - |
| MCF7 | - | - |

| Cancer of pancreas | | |
|---|---|---|
| HPAF-2 | - | - |
| BXPC-3 | - | - |
| Capan-1 | - | - |
| Capan-2 | - | - |

| Melanoma | | |
|---|---|---|
| G361 | - | - |
| Sk-Mel-28 | - | - |

| Neuroblastoma | | |
|---|---|---|
| NAGAI | - | - |
| YT-nu | - | - |
| Glioblastoma | | |
| A172 | - | - |
| T98G | - | - |

| Leukemia | | |
|---|---|---|
| Jurkat (T) | ++ | ++ |
| U-937 (Monocyte) | - | - |
| HL-60 (Promyelo) | - | - |
| HSB-2 (T) | ++ | ++ |
| TALL-1 (T) | - | - |
| KOPN-K (Null) | + | + |
| NALL-1 (Null) | - | - |
| HEL92.1.7 (Erythro) | - | - |
| HPB-ALL (T) | - | - |
| K562 (Myelo) | - | - |
| CCRF-CEM (T) | + | + |
| CCRF-SB (B) | - | - |

| Normal vascular endothelial cell | | |
|---|---|---|
| HUVEC | + | + |

As shown in Fig. 1, the reactivity of the anti-human VEGF receptor Flt-1 monoclonal antibodies KM1730 and KM1732 with the human VEGF receptor-expressing HUVEC-5620 cell is higher than that of the KM1135 control antibody. The reactivity of the anti-human VEGF receptor Flt-1 monoclonal antibodies KM1730 and KM1732 with leukemia cells KOPN-K, CCRE-CEM, HSB-2 and JURKAT is also higher than that of the KM1135 control antibody. On the other hand, the anti-human VEGF receptor Flt-1 monoclonal antibodies KM1730 and KM1732 did not react with the G361 cell and CAPAN-1 cell.

Results of the analysis of a total of 41 cell lines examined in the same manner are summarized in Table 1. Among 12 kinds of the leukemia cells, the KM1730 and KM1732 reacted with 4 cell lines Jurkat, HSB-2, KOPN-K and CCRF-CEM. The reactivities of the anti-human VEGF receptor Flt-1 monoclonal antibodies KM 1730 and KM 1732 with KOPN-K and CCRF-CEM were similar to those of the KM 1730 and KM 1732 with HUVEC, and the reactivities of these monoclonal antibodies with Jurkat and HSB-2 were higher than those with HUVEC. Among the reacted four kinds of leukemia cells, three kinds (Jurkat, HSB-2 and CCRF-CEM) are T cell type leukemia and the remaining one (KOPN-K) is a non-T cell and non-B cell type leukemia. Regarding the T cell type leukemia, they reacted at a high ratio with three kinds among the five kinds examined. On the other hand, regarding the epithelial cell-derived tumors pulmonary adenocarcinoma, pulmonary squamous cell carcinoma, large bowel cancer, uterine cancer, ovarian cancer, gastric cancer, breast cancer and cancer of pancreas and the neurectoderm system-derived tumors pulmonary small cell carcinoma, melanoma, neuroblastoma and glioblastoma, a total of 24 cancer cell lines were examined but the anti-VEGF receptor Flt-1 monoclonal antibodies KM1730 and KM1732 did not react with them.

### INDUSTRIAL APPLICABILITY

The present invention provides a diagnostic agent and a therapeutic agent for leukemia using a monoclonal antibody which specifically binds to the human VEGF receptor Flt-1.

## Claims

1. A diagnostic agent for leukemia, comprising an anti-human VEGF receptor Flt-1 antibody as an active ingredient.

2. The diagnostic agent according to claim 1, wherein the anti-human VEGF receptor Flt-1 antibody is a monoclonal antibody.

3. The diagnostic agent according to claim 1, wherein the anti-human VEGF receptor Flt-1 antibody is an antibody selected from the group consisting of KM1730, KM1731, KM1732, KM1748 and KM1750.

4. The diagnostic agent according to claim 1, wherein the anti-human VEGF receptor Flt-1 antibody is a humanized antibody.

5. The diagnostic agent according to claim 1, wherein the anti-human VEGF receptor Flt-1 antibody is an antibody selected from the group consisting of Fab, Fab', F(ab')₂, a single chain antibody and a disulfide stabilized antibody.

6. The diagnostic agent according to claim 1, wherein the anti-human VEGF receptor Flt-1 antibody is an antibody fused with a radioisotope, a protein or a low molecular weight agent by a chemical or genetic engineering means.

7. A therapeutic agent for leukemia, comprising an anti-human VEGF receptor Flt-1 antibody as an active ingredient.

8. The therapeutic agent according to claim 7, wherein the anti-human VEGF receptor Flt-1 antibody is a monoclonal antibody.

9. The therapeutic agent according to claim 7, wherein the anti-human VEGF receptor Flt-1 antibody is an antibody selected from the group consisting of KM1730, KM1731, KM1732, KM1748 and KM1750.

10. The therapeutic agent according to claim 7, wherein the anti-human VEGF receptor Flt-1 antibody is a humanized antibody.

11. The therapeutic agent according to claim 7, wherein the anti-human VEGF receptor Flt-1 antibody is an antibody selected from the group consisting of Fab, Fab', F(ab')₂, a single chain antibody and a disulfide stabilized antibody.

12. The therapeutic agent according to claim 7, wherein the anti-human VEGF receptor Flt-1 antibody is an antibody fused with a radioisotope, a protein or a low molecular weight agent by a chemical or genetic engineering means.

13. A method for diagnosing leukemia, comprising using an anti-human VEGF receptor Flt-1 antibody.

14. The method for diagnosing leukemia according to claim 13, wherein the anti-human VEGF receptor Flt-1 antibody is a monoclonal antibody.

15. The method for diagnosing leukemia according to claim 13, wherein the anti-human VEGF receptor Flt-1 antibody is an antibody selected from the group consisting of KM1730, KM1731, KM1732, KM1748 and KM1750.

16. The method for diagnosing leukemia according to claim 13, wherein the anti-human VEGF receptor Flt-1 antibody is a humanized antibody.

17. The method for diagnosing leukemia according to claim 13, wherein the anti-human VEGF receptor Flt-1 antibody is an antibody selected from the group consisting of Fab, Fab', F(ab')₂, a single chain antibody and a disulfide stabilized antibody.

18. The method for diagnosing leukemia according to claim 13, wherein the anti-human VEGF receptor Flt-1 antibody is an antibody fused with a radioisotope, a protein or a low molecular weight agent by a chemical or genetic engineering means.
